# FASCICULE DE BREVET EUROPEEN

(11) **EP 0 524 082 B1**
(45) Date de publication et mention de la délivrance du brevet: **18.10.1995**
(21) Numéro de dépôt: 92402031.6
(22) Date de dépôt: 15.07.1992
(51) Int. Cl.: C07C 35/16, C07C 29/17, C07C 29/76

(54) **Procédé de préparation du cis-inositol, du méso-inositol, de l'épi-inositol et/ou du cis-quercitol éventuellement deutérés**
Verfahren zum Herstellen von gegebenfalls deuteriertem Cis-Inositol, Meso-Inositol, Epi-Inositol und/oder Cis-Quercitol
Process for the preparation of eventually deuterated cis-inositol, meso-inositol, epi-inositol and/or cis-quercitol

(30) Priorité: 16.07.1991 FR 9108958
(43) Date de publication de la demande: 20.01.1993
(73) Titulaire: COMMISSARIAT A L'ENERGIE ATOMIQUE, 75015 Paris (FR)
(72) Inventeur: Odier, Léon, F-38100 Grenoble (FR)
(74) Mandataire: Des Termes, Monique

(56) Documents cités:
- US-A- 4 482 761
- CARBOHYDRATE RESEARCH. vol. 183, 1988, AMSTERDAM NL pages 1 - 9 KEN SASAKI 'SEPARATION OF EIGHT INOSITOL ISOMERS BY LIQUID CHROMATOGRAPHY UNDER PRESSURE USING A CALCIUM-FORM,CATION EXCHANGE COLUMN'
- JOURNAL OF THE CHEMICAL SOCIETY. Août 1957, LETCHWORTH GB pages 3682 - 3691 S.J.ANGYAL 'CYCLITOLS.PART VI.tHE HYDROGENATION OF HEXAHYDROXYBENZENE'

## Description

La présente invention a pour objet un procédé de préparation du cis-inositol qui permet en outre d'obtenir du méso-inositol, de l'épi-inositol et du cis-quercitol.

Elle concerne également l'obtention de ces composés entièrement deutérés ainsi que l'utilisation du cis-inositol, deutéré ou non, pour préparer du cis-inositol, de l'épi-inositol et/ou du cis-quercitol spécifiquement deutérés sur des atomes de carbone particuliers.

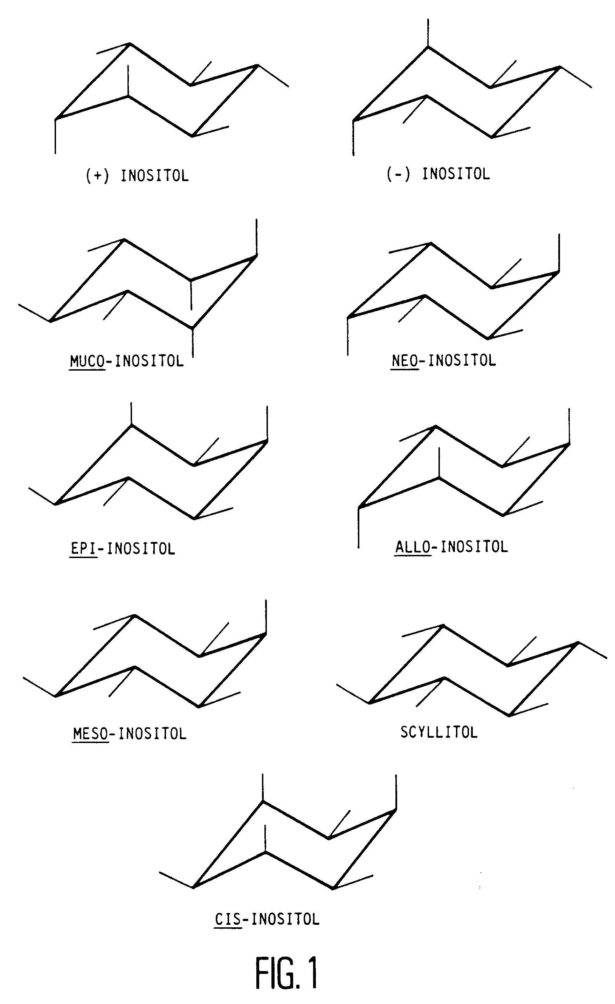

Le cis-inositol est l'un des isomères du cyclohexanehexol de formule : 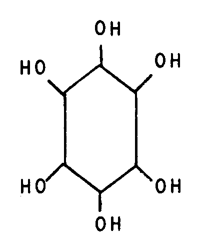
Dans cette formule, on a six groupes hydroxyle qui peuvent être disposés de part et d'autre du plan moyen de la molécule. Si l'on tient compte de tous les arrangements possibles, on constate donc que le cyclohexanehexol peut exister sous neuf formes diastéréoisomères dont deux seulement sont optiquement actives et énantiomères. Ces diastéréoisomères sont représentés sur la figure 1 annexée dans leur forme chaise.

Le premier inositol connu fut découvert par Scherer en 1850 qui l'isola d'extraits de viande d'où son nom de myo-inositol (méso-inositol). Dans les quelques années qui suivirent, sept autres inositols étaient décrits, soit préparés par synthèse, soit isolés à partir de substances naturelles. Un seul des diastéréoisomère était alors manquant, il s'agissait du cis-inositol qui n'avait jamais pu être obtenu malgré un grand nombre de tentatives pour préparer ce composé, et qui n'avait pas non plus été découvert dans la nature. Au cours des décennies qui suivirent, toutes les tentatives pour préparer ce composé se soldèrent par un échec et il fallut attendre 1957 pour qu'Angyal obtienne enfin ce composé par un procédé comprenant une hydrogénation catalytique de l'hexahydroxybenzène ou de la tétrahydroxybenzoquinone, suivie d'une séparation des produits obtenus par chromatographie sur poudre de cellulose et cristallisation fractionnée, comme il est décrit dans J. Chem. Soc., 1957, p. 3682-3691.

Avec ce procédé, la quantité de cis-inositol produite est très faible (rendement de 1,7%) et l'isolement du cis-inostol par chromatographie sur poudre de cellulose est long et difficile à réaliser. On peut toutefois améliorer le rendement de la réaction d'hydrogénation en utilisant un catalyseur palladium-carbone, mais l'emploi d'un tel catalyseur ne peut être envisagé pour la production de cis-inositol à une échelle dépassant celle du laboratoire, en raison du prix élevé de ce catalyseur qui est perdu après la réaction d'hydrogénation. Par ailleurs, la séparation du cis-inositol des produits de la réaction nécessite toujours plusieurs étapes de chromatographie sur la colonne de cellulose.

On connaît un autre procédé de préparation du cis-inositol décrit par Angyal et al dans Carbohydr. Res., 20, 1971, p. 97-104. Ce procédé qui consiste à synthétiser le cis-inositol par épimérisation de l'épi-inositol, permet d'atteindre un rendement supérieur et conduit au seul diastéréoisomère cis sans poser de problème de séparation d'isomères. Cependant, cette synthèse nécessite huit étapes, ce qui est beaucoup trop long et trop coûteux, d'autant plus que le produit de départ, l'épi-inositol, est lui-même un produit cher d'un accès difficile.

On connaît également (Sasaki K. et al., Carbohydr. Res., 183, 1988, 1-9.) une méthode de séparation par chromatographie liquide, sur résine échangeuse de cations, sous forme Ca⁺, d'isomères de l'inositol présents dans le mélange de composés obtenu par traitement du myo-inositol (méso-inositol) par l'eau bouillante en présence de nickel de Raney. La chromatographie se fait sous pression, en utilisant l'eau comme éluant.

Ainsi, aucun des procédés connus ne permet de produire, à un coût raisonnable, du cis-inositol en quantité suffisante étant donné la demande croissante en cis-inositol, notamment dans le domaine pharmaceutique.

La présente invention a précisément pour objet un procédé de préparation du cis-inositol qui permet d'obtenir des rendements intéressants en étant plus facile à mettre en oeuvre et moins coûteux que les procédés décrits ci-dessus.

Selon l'invention, le procédé de préparation du cis-inositol se caractérise en ce que l'on soumet à une hydrogénation catalytique de la tétrahydroxybenzoquinone en utilisant un catalyseur à base de palladium et en ce que l'on récupère ensuite le cis-inositol en séparant les produits obtenus lors de la réaction d'hydrogénation, par chromatographie sur une résine échangeuse de cations comportant des cations bivalents ou trivalents ayant un rayon ionique supérieur à 0,6Å.

A titre d'exemples de tels cations, on peut citer les cations de métaux alcalino-terreux, d'yttrium et de lanthanides, comme La³⁺, Eu³⁺, Pr³⁺, ainsi que Mn²⁺, Fe³⁺, Ni²⁺, Co²⁺, Cu²⁺, Zn²⁺, et Mg²⁺.

Ce procédé peut être également mis en oeuvre pour produire du cis-inositol complètement deutéré en utilisant du deutérium au lieu d'hydrogéne pour l'hydrogénation catalytique de la tétrahydroxybenzoquinone.

La réaction d'hydrogénation correspond au schéma réactionnel suivant : 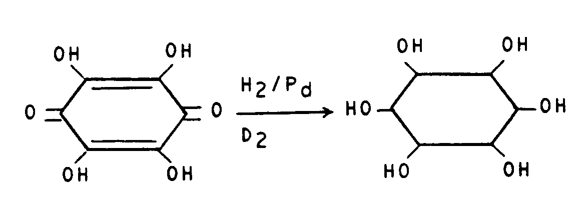
Elle est généralement effectuée en solution aqueuse, à la pression et à la température ambiantes.

Aprés cette réaction, on sépare les différents inositols produits par chromatographie sur une résine échangeuse de cations, en utilisant de l'eau ou une solution aqueuse pour l'élution. Ceci permet de séparer en particulier les isomères suivants : 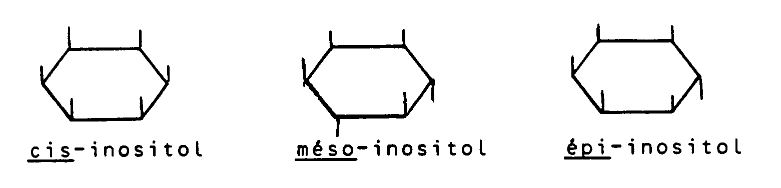
ainsi que du cis-quercitol de formule : 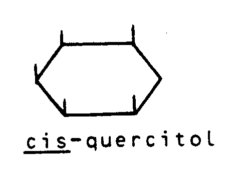
Le cis-inositol étant très retenu par la résine sort dans les dernières fractions, dans un état suffisamment pur pour cristalliser spontanément lorsque l'on concentre les fractions qui le contiennent.

Les résines échangeuses de cations utilisées peuvent être de n'importe quel type, organiques ou inorganiques, et on les met sous la forme voulue par mise en contact avec une solution aqueuse d'un sel du cation choisi.

L'utilisation pour cette chromatographie d'une résine échangeuse de cations comportant des cations bivalents ou trivalents, par exemple de métaux alcalino-terreux, permet donc de séparer facilement le cis-inositol des autres isomères, en raison de la capacité plus élevée du cis-inositol de former des complexes avec les cations, capacité qui est due à sa configuration "tout cis".

De plus, cette séparation par chromatographie peut être effectuée à la température ambiante, en un temps relativement court (1 heure) sans utiliser des matériels sophistiqués tels que les équipements de chromatographie liquide sous haute pression (HPLC).

Les métaux formant les cations de la résine échangeuse de cations sont des métaux facilement complexés par le cis-inositol. Parmi les métaux mentionnés ci-dessus, on peut utiliser en particulier des métaux alcalino-terreux comme le calcium, le strontium et le baryum.

De préférence, on utilise une résine échangeuse de cations sous forme Ca²⁺.

Dans le procédé de l'invention, le fait de partir de la tétrahydroxybenzoquinone est très intéressant car ce produit est beaucoup plus stable que l'hexahydroxybenzène utilisé précédemment.

Par ailleurs, la tétrahydroxybenzoquinone est un produit commercial bon marché que l'on peut obtenir facilement,par exemple par oxydation nitrique du méso-inositol qui est lui-même un produit commercial très bon marché car il est extrait en grande quantité de graines de céréales.

Selon l'invention, on peut de plus améliorer les conditions de la réaction d'hydrogénation, en particulier la vitesse et le rendement de la réaction en utilisant au moins au début de celle-ci un catalyseur constitué de palladium qui a été préréduit en milieu neutre.

En effet, si l'on utilise dans l'invention un catalyseur classique au palladium, c'est-à-dire de l'oxyde-hydroxyde de palladium, qui est réduit "in situ" dans le mélange réactionnel, la réaction d'hydrogénation a lieu plus lentement et son rendement est plus faible. En effet, la tétrahydroxybenzoquinone ayant en solution aqueuse un pH acide, la réduction du palladium en milieu acide conduit à un autre type de catalyseur (palladium gris) qui catalyse la réduction de la tétrahydroxybenzoquinone en hexahydroxybenzène seulement et non en inositols ; de plus l'hexahydroxybenzène est peu soluble dans l'eau, il précipite de ce fait à l'intérieur du catalyseur et le désactive complètement.

Selon l'invention, on évite cette réduction en tétrahydroxybenzène, en utilisant tout au moins au début de la réaction un catalyseur de palladium préréduit en milieu neutre.

De préférence, encore, on réalise l'hydrogénation catalytique en utilisant comme catalyseur, au début de la réaction, du palladium préréduit en milieu neutre, puis de l'oxyde-hydroxyde de palladium réduit "in situ".

En effet, lorsque la tétrahydroxyquinone a complètement disparu du mélange réactionnel, il n'est plus nécessaire d'utiliser du palladium préréduit en milieu neutre, et on préfère alors poursuivre la réaction avec du palladium réduit "in situ" dans le milieu réactionnel car ce catalyseur est plus actif que le palladium préréduit.

Le procédé de préparation du cis-inositol de l'invention est donc particulièrement avantageux car il est simple, rapide, facile à mettre en oeuvre et peu coûteux. Il ne nécessite ni réactifs chers, ni équipements sophistiqués ; il ne présente pas de danger particulier et n'utilise aucun produit toxique.

En effet, le produit le plus cher est le palladium, mais il n'est pas consommé, peut donc être récupéré en fin de réaction et réutilisé. Il en est de même pour la résine échangeuse de cations qui peut être réutilisée indéfiniment.

De plus, ce procédé permet d'obtenir, facilement des composés complètement deutérés.

D'autres caractéristiques et avantages de l'invention apparaîtront mieux à la lecture de la description qui suit d'exemples de réalisation de l'invention donnés bien entendu à titre illustratif et non limitatif.

La figure 1 déjà décrite représente les 9 diastéréoisomères de l'inositol.

les figures 2 à 5 représentent diverses réactions qui peuvent être mises en oeuvre à partir du cis-inositol obtenu par le procédé de l'invention.

### Exemple 1 : Préparation de cis-inositol.

### 1) Hydrogénation catalytique.

### a) Préparation du catalyseur.

On dissout à chaud, dans un mélange de 14ml d'acide nitrique concentré et de 9ml d'acide chlorhydrique concentré ( 11N), 3g de palladium métallique sous forme de fil, de plaquette ou de mousse. On évapore ensuite la solution à sec et on dissout le résidu rouge brun de chlorure de palladium (3,5g) dans 18ml d'acide chlorhydrique concentré ( 11N).

On dilue la solution à 1800ml avec de l'eau distillée et on y ajoute lentement 46ml d'une solution de soude à 40%. On porte à l'ébullition la solution jaune ainsi obtenue. Il se forme un léger précipité brun qui se décante lentement, et on sépare celui-ci de la solution par centrifugation car le précipité est si ténu qu'il passe à travers tous les filtres, mais cet état de division est nécessaire à l'activité du catalyseur.

On lave plusieurs fois le précipité à l'eau bouillante jusqu'à ce que les eaux de lavage soient neutres (même pH que l'eau distillée) en réalisant une centrifugation entre chaque lavage pour récupérer le précipité.

On met le précipité (mélange d'oxyde et d'hydroxyde de palladium) en suspension dans de l'eau distillée et on le réduit par l'hydrogéne à la pression atmosphérique en poursuivant l'hydrogénation jusqu'à ce que l'absorption d'hydrogène cesse (environ 18h). Au cours de la réduction, la suspension de palladium redevient basique. On lave alors le palladium réduit (palladium noir) à l'eau distillée jusqu'à ce que la liqueur surnageante soit neutre (pH identique à celui de l'eau distillée).

On obtient ainsi du palladium préréduit en milieu neutre qui doit être utilisé le plus rapidement possible car son activité décroît avec le temps. On conserve celui-ci dans l'eau distillée car son activité diminue si l'eau est évaporée à sec.

### b) Hydrogénation de la tétrahydroxybenzoquinone.

Dans une suspension de 2g du palladium préréduit obtenu précédemment dans 100ml d'eau distillée, on introduit goutte à goutte une solution de 3g de tétrahydroxybenzoquinone (en partie dissoute) dans 250ml d'eau distillée, sous atmosphère d'hydrogéne, en agitant vigoureusement. Après introduction de la tétrahydroxybenzoquinone, on poursuit l'hydrogénation jusqu'à ce que l'absorption d'hydrogène cesse. On ajoute alors une nouvelle quantité de catalyseur correspondant à environ 1g de palladium sous forme d'oxyde, c'est-à-dire 1g du précipité brun obtenu précédemment, et on poursuit l'hydrogénation jusqu'à ce que l'absorption d'hydrogène cesse (environ 10h).

On sépare alors le palladium de la solution ; à ce stade, il n'est plus pulvérulent, il s'est aggloméré en particules plus grosses et peut être séparé aisément par filtration ou centrifugation. On le lave trois fois à l'eau bouillante et on joint les eaux de lavage au filtrat. Si la solution est encore trouble, on peut la clarifier par filtration sur un filtre millipore de 0,5µm. On évapore ensuite la solution sous pression réduite, ce qui donne 2,9g d'un résidu semicristallin incolore.

### 2. Séparation par chromatographie.

### a) Préparation de la colonne de chromatographie.

On introduit dans une colonne de chromatographie de 2,5cm de diamètre, une suspension dans une solution de CaCl₂ à 20% d'une résine échangeuse de cations, commercialisée sous la marque DOWEX AG 50W-X2 de 100-200mesh (0,152 à 0,076mm) sous forme hydrogène, en quantité suffisante pour que la colonne de résine atteigne une hauteur de 10cm environ après décantation de la résine. On lave ensuite la colonne de résine par une solution de CaCl₂ à 20% en quantité égale à 2fois son volume, puis on la lave à l'eau distillée jusqu'à ce que l'eau de lavage à la sortie de la colonne ne contienne plus d'ions Ca²⁺. On vérifie l'absence d'ions Ca²⁺ dans les eaux de lavage à l'aide d'une solution de CO₃Na₂ qui, normalement précipite du CO₃Ca en présence d'ions calcium.

### b) Chromatographie.

On dissout les 2,9g du résidu semicristallin incolore obtenu précédemment dans l'étape 1.b) dans 20ml d'eau distillée et on introduit cette solution au sommet de la colonne. Lorsque la solution a entièrement pénétré dans la colonne, on élue celle-ci par de l'eau distillée et on collecte à la sortie de la colonne des fractions de 15ml. On détecte dans les fractions recueillies, la présence du cis-inositol, de l'épi-inositol, du cis-quercitol et des autres inositols par chromatographie sur couche mince sur gel de silice sans réaliser d'élution. On récupère les produits obtenus dans chaque fraction par cristallisation en concentrant les fractions.

Les produits obtenus sont répartis dans les fractions de la façon suivante :
- fractions 3 à 9 :: 1,2g d'un mélange d'inositols dont le méso (majoritaire)
- fractions 10 à 13 :: pas de produits
- fractions 14 à 18 :: 0,8g d'un mélange d'épi-inositol et de cis-quercitol
- fractions 19 à 60 :: pas de produits
- fractions 61 à 70 :: 0,9g de cis-inositol pur.

On obtient ainsi 0,9g de cis-inositol pur que l'on recristallise dans un mélange eau-éthanol, ce qui correspond à un rendement de 30%.

On identifie le cis-inositol par mesure de son point de fusion et par spectrométrie à résonance magnétique nucléaire (RMN).

### Point de fusion.

On observe une fusion pâteuse à 375-390°C, qui correspond à la destruction progressive des liaisons hydrogène. Ce point de fusion très caractéristique car il est anormalement élevé pour un composé organique, est dû au fait que les molécules sont fortement liées par des liaisons hydrogène intermoléculaires, conséquence de la configuration "tout "cis", formant ainsi des édifices cristallins très stables.

Si l'on transforme le cis-inositol produit en hexaacétate par réaction avec l'anhydride acétique dans la pyridine, on observe une fusion franche à 210-212°C caractéristique de cet hexaacétate de cis-inositol.

### Spectrométrie RMN.

A la température ambiante, le spectre est très caractéristique car il présente une seule raie extrêmement large due à l'inversion de la forme chaise, trop rapide pour pouvoir séparer les protons axiaux et équatoriaux, trop lente pour que ces protons soient équivalents.

Si l'on refroidit à -2°C, la conformation ne s'inverse plus et on observe deux signaux, l'un pour les protons axiaux et l'autre pour les protons équatoriaux.

Si l'on chauffe à 80°C, la conformation s'inverse rapidement, ce qui rend les protons équivalents et l'on observe une seule raie fine.

### Exemple 2 : Préparation de cis-inositol deutéré.

On procède comme dans l'exemple 1, sauf que l'on utilise du deutérium pour réaliser dans l'étape 1a) la réduction du palladium et dans l'étape 1b) d'hydrogénation catalytique de la tétrahydroxybenzoquinone.

Dans ces conditions, on obtient également 0,9g de cis-inositol complètement deutéré, c'est-à-dire dans lequel subsistent uniquement les atomes d'hydrogène des groupements hydroxyle, tous les autres atomes d'hydrogène étant des atomes de deutérium.

On obtient également du méso-inositol, de l'épi-inositol et du cis-quercitol complètement deutérés.

Le cis-inositol obtenu dans l'exemple 1 peut être utilisé pour préparer du cis-inositol, de l'épi-inositol ou du cis-quercitol partiellement et spécifiquement deutérés en utilisant des réactions connues.

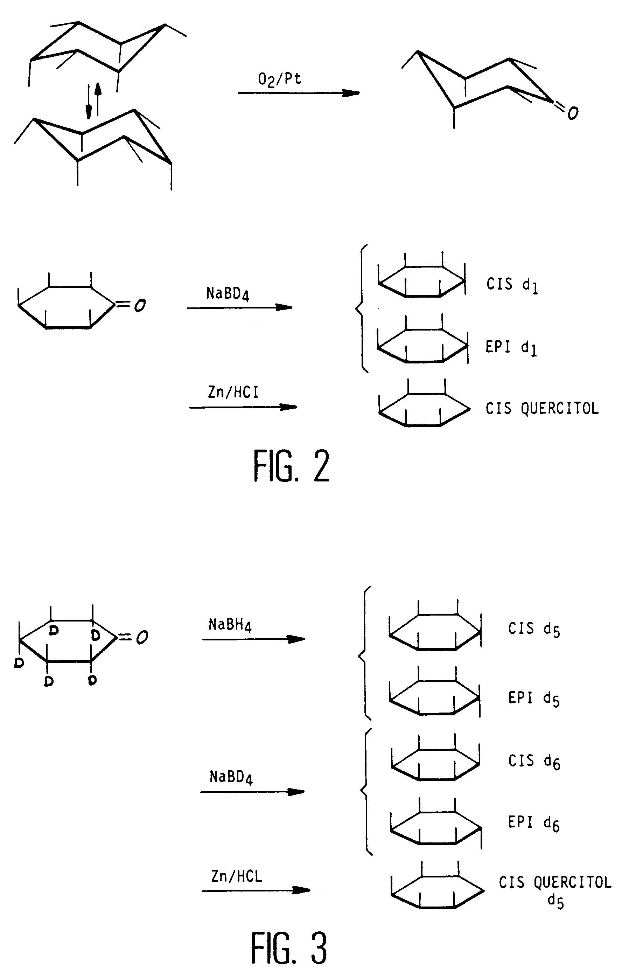

Ainsi, on peut obtenir du cis-inositol ou de l'épi-inositol sélectivement deutéré sur une seule position en soumettant le cis-inositol à une oxydation ménagée pour produire une monocétone que l'on réduit ensuite par un réactif deutéré tel que NaBD₄, ce qui conduit au cis ou à l'épi-inositol sélectivement deutéré. Ceci correspond au schéma donné sur la figure 2.

Le cis-inositol conduit à une seule monocétone car les groupes hydroxyle du cis-inositol sont disposés sur des liaisons alternativement axiale et équatoriale, si bien qu'il existe sous une seule conformation chaise qui s'inverse rapidement à la température ambiante rendant ainsi tous les hydroxyle équivalents.

A partir de la monocétone, on peut aussi produire du cis-quercitol par réaction avec de l'acide chlorhydrique en présence de zinc. Le schéma réactionnel est illustré également sur la figure 2.

On peut effectuer le même type de réaction en partant du cis-inositol complètement deutéré, en le soumettant à une oxydation ménagée pour obtenir la monocétone complètement deutérée que l'on réduit ensuite par un réactif hydrogéné tel que NaBH₄ pour obtenir le cis et l'épi-inositol contenant un seul proton qui sont intéressants comme produits marqués, notamment en RMN. Si l'on réalise la réaction de réduction avec NaBD₄ on obtient le cis ou l'épi-inositol complètement deutéré. Ces réactions sont illustrées sur la figure 3.

De la même façon, on peut produire du cis-quercitol complètement deutéré par réaction avec l'acide chlorhydrique en présence de zinc. Le schéma réactionnel est donné également sur la figure 3.

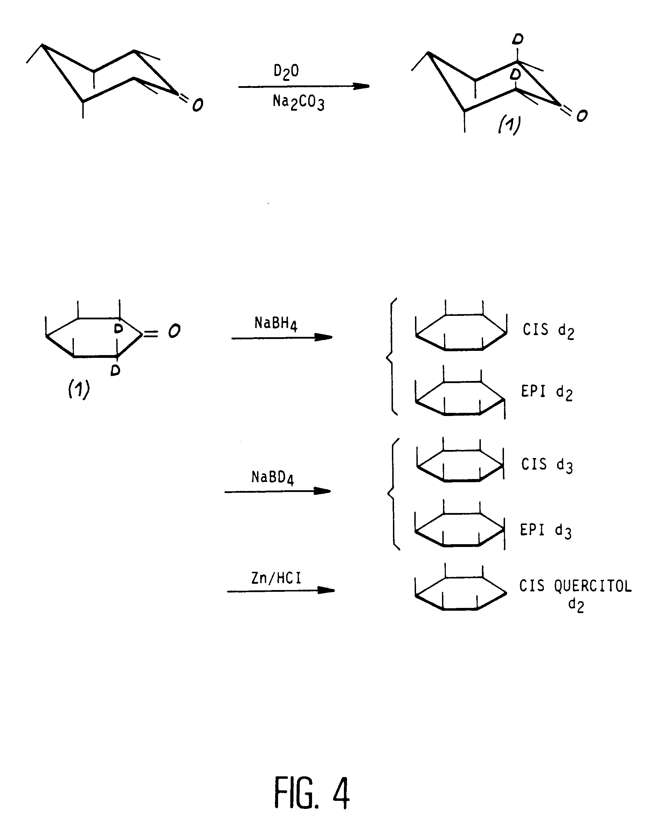

On peut aussi faire réagir la monocétone avec de l'eau lourde en milieu légèrement basique pour échanger les deux protons en alpha et obtenir ainsi à partir de cette monocétone des inositols et du quercitol sélectivement marqués sur deux ou trois positions comme il est indiqué sur les schémas réactionnels de la figure 4 qui font appel aux mêmes réactions que celles des figures 2 et 3.

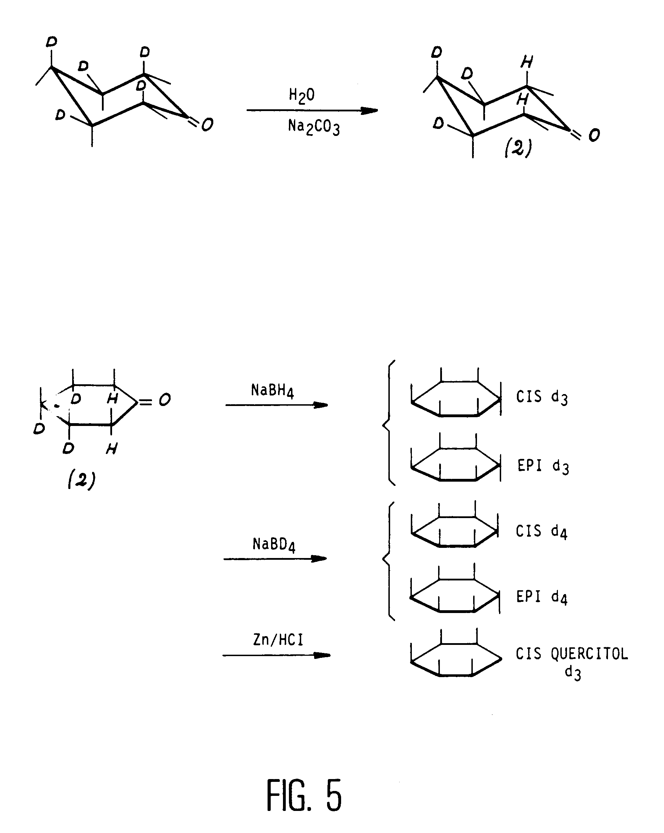

Si l'on effectue les mêmes réactions en partant de la monocétone complètement deutérée et en la soumettant à un échange avec de l'eau en milieu légèrement basique, on obtiendra des inositols ou du quercitol comportant trois ou quatre atomes de deutérium. Ces réactions sont données sur la figure 5.

## Revendications

1. Procédé de préparation du cis-inositol, caractérisé en ce que l'on soumet à une hydrogénation catalytique de la tétrahydroxybenzoquinone en utilisant un catalyseur à base de palladium et en ce que l'on récupère ensuite le cis-inositol en séparant les produits obtenus lors de la réaction d'hydrogénation, par chromatographie sur une résine échangeuse de cations comportant des cations bivalents ou trivalents ayant un rayon ionique supérieur 0,6Å .

2. Procédé de préparation du cis-inositol deutéré, caractérisé en ce que l'on soumet à une hydrogénation catalytique au moyen de deutérium de la tétrahydroxybenzoquinone en utilisant un catalyseur à base de palladium et en ce que l'on récupère ensuite le cis-inositol deutéré en séparant les produits obtenus lors de la réaction d'hydrogénation, par chromatographie sur une résine échangeuse de cations comportant des cations bivalents ou trivalents ayant un rayon ionique supérieur à 0,6Å.

3. Procédé selon l'une quelconque des revendications 1 et 2, caractérisé en ce que la résine échangeuse de cations est sous forme Ca²⁺.

4. Procédé selon l'une quelconque des revendications 1 à 3, caractérisé en ce que l'on réalise l'hydrogénation catalytique en utilisant comme catalyseur du palladium qui a été préréduit en milieu neutre.

5. Procédé selon l'une quelconque des revendications 1 à 3, caractérisé en ce que l'on réalise l'hydrogénation catalytique en utilisant comme catalyseur du palladium préréduit en milieu neutre au début de la réaction, puis de l'oxyde de palladium qui est réduit "in situ".

6. Procédé selon l'une quelconque des revendications 1 à 5, caractérisé en ce que l'on récupère en outre le méso-inositol, l'épi-inositol et/ou le cis-quercitol séparés sur la résine échangeuse de cations.

7. Utilisation du cis-inositol produit par le procédé selon l'une quelconque des revendications 1 à 5 pour la préparation de cis-inositol partiellement deutéré, d'épi-inositol partiellement deutéré ou de cis-quercitol partiellement deutéré.

## Claims

1. Process for the preparation of cis-inositol, characterized in that the latter undergoes a catalytic hydrogenation of the letrahydroxybenzoquinone using a palladium-based catalyst and in that the cis-inositol is then recovered by separating the products obtained during the hydrogenation reaction by chromatography on a cation exchange resin incorporating divalent or trivalent cations having an ionic radius greater than 0.6Å.

2. Process for the preparation of deuterated cis-inositol, characterized in that it undergoes a catalytic hydrogenation by means of deuterium of tetrahydroxybenzoquinone using a palladium-based catalyst and that the deuterated cis-inositol is then recovered by separating the products obtained during the hydrogenation reaction by chromatography on a cation exchange resin incorporating divalent or trivalent cations having an ionic radius greater than 0.6Å.

3. Process according to either of the claims 1 and 2, characterized in that the cation exchange resin is in form Ca²⁺.

4. Process according to any one of the claims 1 to 3, characterized in that the catalytic hydrogenation takes place by using as the catalyst palladium prereduced in a neutral medium.

5. Process according to any one of the claims 1 to 3, characterized in that catalytic hydrogenation is carried out by using as the catalyst palladium prereduced in a neutral medium at the start of the reaction and then palladium oxide reduced in situ.

6. Process according to any one of the claims 1 to 5, characterized in that recovery also takes place of the meso-inositol, epi-inositol and/or cis-quercitol separated on the cation exchange resin.

7. Use of the cis-inositol produced by the process according to any one of the claims 1 to 5 for the preparation of partly deuterated cis-inositol, partly deuterated epi-inositol or partly deuterated cis-quercitol.

## Patentansprüche

1. Verfahren zum Herstellen von cis-Inositol, **dadurch gekennzeichnet,** daß Tetrohydroxybenzochinon einer katalytischen Hydrierung unterworfen wird, wobei ein Katalysator auf Palladium-Basis verwendet wird, und daß anschließend das cis-Inositol durch Trennen der bei der Hydrierungsreaktion erhaltenen Produkte durch Chromatographie an einem Kationenaustauscherharz, welches zweiwertige oder dreiwertige Kationen mit einem Ionenradius von mehr als 0,6 Å umfaßt, gewonnen wird.

2. Verfahren zum Herstellen von deuteriertem cis-Inositol, **dadurch gekennzeichnet,** daß Tetrahydroxybenzochinon einer katalytischen Hydrierung mit Deuterium unterworfen wird wobei ein Katalysator auf Palladium-Basis verwendet wird, und daß anschließend das deuterierte cis-Inositol durch Trennen der bei der Hydrierungsreaktion erhaltenen Produkte durch Chromatographie an einem Kationenaustauscherharz, welches zweiwertige oder dreiwertige Kationen mit einem Ionenradius von mehr als 0,6 Å umfaßt, gewonnen wird.

3. Verfahren nach einem der Ansprüche 1 und 2, **dadurch gekennzeichnet,** daß das Kationenaustauscherharz in der Ca²⁺-Form vorliegt.

4. Verfahren nach einem der Ansprüche 1 bis 3, **dadurch gekennzeichnet,** daß die katalytische Hydrierung unter Verwendung von Palladium, welches in neutraler Umgebung vorreduziert worden ist, als Katalysator durchgeführt wird.

5. Verfahren nach einem der Ansprüche 1 bis 3, **dadurch gekennzeichnet,** daß die katalytische Hydrierung unter Verwendung von in neutraler Umgebung vorreduziertem Palladium zu Beginn der Reaktion, und anschließend von Palladiumoxid, welches *in situ* reduziert wird, als Katalysator durchgeführt wird.

6. Verfahren nach einem der Ansprüche 1 bis 5, **dadurch gekennzeichnet,** daß außerdem meso-Inositol, epi-Inositol und/oder cis-Quercitol gewonnen werden, die an dem Kationenaustauscherharz getrennt werden.

7. Verwendung von cis-Inositol, welches durch das Verfahren nach einem der Ansprüche 1 bis 5 hergestellt ist, zum Herstellen von teilweise deuteriertem cis-Inositol, teilweise deuteriertem epi-Inositol oder teilweise deuteriertem cis-Quercitol.
